# EUROPEAN PATENT APPLICATION

(11) **EP 4 379 739 A1**
(43) Date of publication of application: **05.06.2024**
(21) Application number: 22849678.2
(22) Date of filing: 06.05.2022
(51) Int. Cl.: G16H 50/50, G16H 50/70, G16H 10/60, G16H 30/40, G16H 30/20, G06N 20/00

(54) **METHOD AND SYSTEM FOR GENERATING INTERPRETABLE PREDICTION RESULT FOR PATIENT**

(30) Priority: 30.07.2021 KR 20210100989
(71) Applicant: Lunit Inc., Seoul 06241 (KR)
(72) Inventor: AHN, Jong Seok, Seoul 06241 (KR); LEE, Jeong Hoon, Seoul 06241 (KR)
(74) Representative: AWA Sweden AB
(86) International application number: PCT/KR2022/006463
(87) International publication number: WO 2023/008699

(57) **Abstract**

Provided is a method, performed by at least one computing apparatus, of generating an interpretable prediction result for a patient. The method includes receiving medical image data of a subject patient, receiving additional medical data of the subject patient, and generating information about a prediction result for the subject patient, based on the medical image data of the subject patient and the additional medical data of the subject patient, by using a machine learning prediction model.

## Description

### Technical Field

The disclosure relates to a method and system for generating an interpretable prediction result for a patient, and more particularly, to a method and apparatus for generating a prediction result for a subject patient based on medical image data and additional medical data about the subject patient by using a machine learning prediction model.

### Background Art

A deep learning model corresponds to a complex model that may receive multidimensional data, and has made great progress in that feature selection and prediction may be simultaneously performed. For example, due to the development of deep learning technology, it has been possible to develop methodologies to construct a system for predicting an intervention and a prognosis for a patient based on medical images, such as X-ray, computed tomography (CT), and magnetic resonance imaging (MRI) scans, from a patient with COVID-19, infectious diseases, lung cancer, or various other lung-related diseases.

However, predicting future events, such as a prognosis and an intervention for a patient, is performed by an uninterpretable black-box system, and thus, direct grounds for prediction results may not be provided to a medical staff. When only predicted result values for the prognosis and the intervention for the patient are provided, the result values may not be very helpful for the medical staff to make a clinical determination about the patient, and rather may aggravate confusion and difficulties.

### Detailed Description of the Invention

### Technical Problem

In order to solve the technical problem as described above, the disclosure provides a method and system for generating an interpretable prediction result for a patient.

### Technical Solution to Problem

Additional aspects will be set forth in part in the description which follows and, in part, will be apparent from the description, or may be learned by practice of the presented embodiments of the disclosure.

The disclosure may be implemented in various forms including a method, an apparatus (system), a computer-readable storage medium storing instructions, or a computer program.

According to an embodiment of the disclosure, a method, performed by at least one computing apparatus, of generating an interpretable prediction result for a patient includes receiving medical image data of a subject patient, receiving additional medical data of the subject patient, and generating information about a prediction result for the subject patient, based on the medical image data of the subject patient and the additional medical data of the subject patient, by using a machine learning prediction model.

According to an embodiment of the disclosure, the method may further include generating information about a factor affecting generation of the information about the prediction result for the subject patient, by using the machine learning prediction model.

According to an embodiment of the disclosure, the method may further include providing, to a user terminal, at least one of the information about the prediction result for the subject patient or the information about the factor.

According to an embodiment of the disclosure, the machine learning prediction model may include a first sub-prediction model and a second sub-prediction model, and the generating of the information about the prediction result for the subject patient may include extracting one or more features from the medical image data of the subject patient, by using the first sub-prediction model, and generating the information about the prediction result for the subject patient, based on the one or more features and the additional medical data of the subject patient, by using the second sub-prediction model.

According to an embodiment of the disclosure, the method may further include generating information about a factor affecting generation of the information about the prediction result for the subject patient, by using the machine learning prediction model, wherein the generating of the information about the factor may include obtaining information about an importance of each of a plurality of factors in generating the information about the prediction result for the subject patient, by using the second sub-prediction model, wherein the plurality of factors may include at least one of the additional medical data of the subject patient or the one or more features.

According to an embodiment of the disclosure, the generating of the information about the factor may further include determining at least one of the plurality of factors as a prediction reason, based on the information about the importance.

According to an embodiment of the disclosure, the one or more features may include an interpretable phenotypic feature that is usable to interpret the information about the prediction result for the subject patient.

According to an embodiment of the disclosure, the first sub-prediction model may be trained to extract one or more reference features from medical image data of a reference patient, and the second sub-prediction model may be trained to generate reference information about a reference prediction result for the reference patient, based on additional medical data of the reference patient and the one or more reference features.

According to an embodiment of the disclosure, the generating of the information about the prediction result for the subject patient, based on the one or more features and the additional medical data of the subject patient, by using the second sub-prediction model may include generating input data of the second sub-prediction model by concatenating the additional medical data of the subject patient with the one or more features, and generating the information about the prediction result for the subject patient by inputting the generated input data to the second sub-prediction model.

According to an embodiment of the disclosure, the additional medical data of the subject patient may include at least one of clinical data, lab data, or biological data of the subject patient.

According to an embodiment of the disclosure, provided is a computer program stored in a computer-readable recording medium for executing, on a computer, the method of generating the interpretable prediction result for the patient.

According to an embodiment of the disclosure, a computing apparatus includes a memory storing one or more instructions, and a processor configured to execute the one or more stored instructions to receive medical image data of a subject patient, receive additional medical data of the subject patient, and generate information about a prediction result for the subject patient, based on the medical image data of the subject patient and the additional medical data of the subject patient, by using a machine learning prediction model.

### Brief Description of Drawings

The above and other aspects, features, and advantages of certain embodiments of the disclosure will be more apparent from the following description taken in conjunction with the accompanying drawings.

Embodiments of the disclosure will be described with reference to the accompanying drawings described below, and like reference numerals denote like elements but are not limited thereto.
FIG. 1 illustrates a system in which an information processing system provides an interpretable prediction result for a patient, according to an embodiment of the disclosure.
FIG. 2 is a block diagram illustrating an internal configuration of an information processing system, according to an embodiment of the disclosure.
FIG. 3 is a flowchart illustrating a method of generating an interpretable prediction result for a patient, according to an embodiment of the disclosure.
FIG. 4 illustrates an example of generating information about a prediction result for a patient and information about a factor affecting generation of the information about the prediction result by using a machine learning prediction model, according to an embodiment of the disclosure.
FIG. 5 illustrates an example of generating information about a prediction result for a patient and information about a factor affecting generation of the information about the prediction result by using a first sub-prediction model and a second sub-prediction model, according to an embodiment of the disclosure.
FIG. 6 illustrates an example of extracting one or more features from medical image data by using a convolutional neural network (CNN)-based first sub-prediction model, according to an embodiment of the disclosure.
FIG. 7 illustrates an example of generating information about a prediction result for a patient by using a second sub-prediction model, according to an embodiment of the disclosure.
FIG. 8 is a table illustrating prediction performance when prediction is performed on a patient, according to an embodiment of the disclosure.
FIG. 9 is a table illustrating impacts of a plurality of factors in performing prediction on a subject patient, according to an embodiment of the disclosure.
FIG. 10 is an exemplary diagram illustrating an artificial neural network model according to an embodiment of the disclosure.
FIG. 11 is a block diagram of a computing apparatus (e.g., an information processing system) for generating an interpretable prediction result for a patient, according to an embodiment of the disclosure.

### Best Mode

According to an embodiment of the disclosure, a method, performed by at least one computing apparatus, of generating an interpretable prediction result for a patient includes receiving medical image data of a subject patient, receiving additional medical data of the subject patient, and generating information about a prediction result for the subject patient, based on the medical image data of the subject patient and the additional medical data of the subject patient, by using a machine learning prediction model.

### Mode of Disclosure

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

Hereinafter, specific embodiments of the disclosure will be described in detail with reference to the accompanying drawings. However, in the following descriptions, when there is a risk of unnecessarily obscuring the gist of disclosure, detailed descriptions of well-known functions or configurations will be omitted.

In the accompanying drawings, the same or corresponding components are denoted by the same reference numerals. In addition, in the following descriptions of the embodiments, redundant descriptions of the same or corresponding components may be omitted. However, even though descriptions regarding components are omitted, it is not intended that such components are not included in any of the embodiments.

The advantages and features of the disclosure and methods of achieving them will become apparent with reference to embodiments of the disclosure described in detail below with reference to the accompanying drawings. The disclosure may, however, be embodied in many different forms and should not be construed as being limited to the embodiments set forth herein. Rather, these embodiments are provided so that the disclosure will be thorough and complete, and will fully convey the concept of the disclosure to one of ordinary skill in the art.

Hereinafter, the terms used herein will be briefly described, and then embodiments of the disclosure will be described in detail. All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, specified terms may be arbitrarily selected by the applicant, and in this case, the detailed meaning thereof will be described in the detailed description of the disclosure. Thus, the terms used herein have to be defined based on the meaning of the terms together with the description throughout the disclosure.

As used herein, the singular forms "a", "an", and "the" are intended to include the plural forms as well, unless the context clearly indicates the singular forms. The plural forms are also intended to include the singular forms as well, unless the context clearly indicates the plural forms. Throughout the specification, when a portion 'includes' a component, another component may be further included, rather than excluding the existence of the other component, unless otherwise described.

In addition, the term 'module' or '...er/or used herein refers to a software or hardware component, and performs certain tasks. However, the term 'module' or '...er/or' is not limited to software or hardware. The term 'module' or '...er/or' may be configured in an addressable storage medium or may be configured to reproduce one or more processors. Thus, for example, the term 'module' or '...er/or' may include at least one of components (e.g., software components, object-oriented software components, class components, and task components), processes, functions, attributes, procedures, subroutines, segments of program code, drivers, firmware, micro-codes, circuits, data, a database, data structures, tables, arrays, or variables. Functions provided in components and 'modules' or '...ers/ors' may be combined with fewer components and 'modules' or '...ers/ors' or may be divided into more components and modules' or '...ers/ors'.

According to an embodiment of the disclosure, a 'module' or '...er/or' may be implemented as a processor or a memory. The 'processor' should be construed broadly to include a general-purpose processor, a central processing unit (CPU), a microprocessor, a digital signal processor (DSP), a controller, a microcontroller, and a state machines. In some circumstances, the 'processor' may refer to an application specific semiconductor (ASIC), a programmable logic device (PLD), a field programmable gate array (FPGA), or the like. The 'processor' may also refer to a combination of processing apparatuses, for example, a combination of a DSP and a microprocessor, a combination of a plurality of microprocessors, a combination of one or more microprocessors combined with a DSP core, or any other combinations of such components. Also, the 'memory' should be construed broadly to include any electronic components capable of storing electronic information. The 'memory' may also refer to various types of processor-readable media, such as random access memory (RAM), read-only memory (ROM), nonvolatile random access memory (NVRAM), programmable read-only memory (PROM), erase-programmable read-only memory (EPROM), electrically erasable PROM (EEPROM), a flash memory, a magnetic or optical data storage, and registers. When the processor may read information from the memory and/or write information to the memory, the memory may be referred to be in electronic communication with the processor. A memory integrated in the processor is in electronic communication with the processor.

In the disclosure, the term 'system' may include at least one of a server apparatus or a cloud apparatus, but is not limited thereto. For example, the system may include one or more server apparatuses. As another example, the system may include one or more cloud apparatuses. Also, as another example, a server apparatus and a cloud apparatus may constitute the system and operate together.

In the disclosure, an 'interpretable prediction result' may refer to a prediction result and/or information about the prediction result for which a reason or ground for prediction may be presented. For example, the interpretable prediction result may include a prediction result and/or information about the prediction result that may provide information (e.g., the importance of factors) about factors affecting prediction performed by a machine learning prediction model. Similarly, an 'interpretable model', an 'interpretable prediction model', and/or an 'interpretable machine learning prediction model' used herein may refer to a model that may generate (or output) an interpretable prediction result. That is, the interpretable model, the interpretable prediction model, and/or the interpretable machine learning prediction model may correspond to a model capable of presenting a reason or ground for prediction, together with a prediction result.

In the disclosure, 'medical image data' and/or a 'medical image' may refer to an image or a video from which phenotypic features appearing in patients' bodies may be extracted. For example, the medical image data may include image data and/or video data of all modalities, such as chest radiograph, X-ray, computed tomography (CT), positron emission tomography (PET), magnetic resonance imaging (MRI), ultrasound imaging, sonography (ultrasound (US)), functional magnetic resonance imaging (fMRI), digital pathology whole slide image (WSI), mammography (MMG), and digital breast tomosynthesis (DBT).

In the disclosure, 'clinical data' may include all types of information that may be obtained from a patient and recorded. For example, the clinical data may include lab data and biological data. In an embodiment, the clinical data is information that a medical staff may obtain from a patient and record, and may include information (e.g., an address, symptoms, past medical history, family history, and smoking state) obtained through history taking from a patient, physical examination results (e.g., a patient's blood pressure, heart rate, and abdominal examination), and additional test data (e.g., blood test results, electrocardiogram, and serum test). For example, the clinical data may include all pieces of clinical information about a patient, such as age, gender, blood pressure, body temperature, cough, and underlying disease. For example, in the case of a cancer patient, clinical data of the cancer patient may include information about a cancer tumor, node and metastasis (TNM) stage, but is not limited thereto.

In the disclosure, 'biological data' may include genomic data, DNA mutation, copy-number, RNA expression, protein expression, methylation, and microbiome.

In the disclosure, 'information about A' may include A itself, data associated with A, and/or numerical values associated with A. For example, 'information about a prediction result for a patient' may include a 'prediction result for a patient', 'data generated in a process of performing prediction on a patient', and 'accuracy of a prediction result for a patient'. Also, 'information about a factor affecting generation of information about a prediction result' may include a 'factor affecting generation of information about a prediction result', an 'importance indicating an impact of a factor in generating information about a prediction result', and a 'relative importance between factors affecting generation of information about a prediction result'. For example, 'information about factors affecting generation of information about a prediction result' may include information that a factor for fever is 5 times more important than a factor for muscle pain in predicting a patient's prognosis.

In the disclosure, a 'factor' may refer to data input to or data output from at least one of a plurality of layers of a machine learning model. For example, the factor may include input data of the machine learning model, data generated in a process of performing prediction, and information extracted from the input data. In an embodiment, the factor may include a patient's medical image data (e.g. chest X-ray, abdominal CT, and brain MRI), clinical data (e.g., symptoms and signs, such as sore throat, fever, and blood pressure levels, and a patient's questionnaire results), biological data, and lab data (e.g., blood test results, such as white blood cell count and platelet count). Additionally or alternatively, the factor may include information about a surrounding environment (e.g., a medical environment, a treatment environment, a medical infrastructure, and medical resources) to which a patient belongs to.

In the disclosure, 'target data' may refer to arbitrary data or data item that may be used for training the machine learning model. For example, the target data may include, but is not limited to, medical image data and/or additional medical data, and any data that may be used for training the machine learning model may correspond to the target data. Also, the target data may be tagged with label information via an annotation operation.

In the disclosure, a 'machine learning model' and/or an 'artificial neural network model' may include any model used to infer a correct answer to a given input. According to an embodiment, the machine learning model may include an artificial neural network model including an input layer, a plurality of hidden layers, and an output layer. In this case, each layer may include a plurality of nodes. For example, the machine learning model may be trained to output information about a prediction result for a subject patient, based on medical image data and/or additional medical data of the subject patient. As another example, the machine learning model may be trained to extract one or more features from medical image data of the subject patient. Also, as another example, the machine learning model may be trained to output information about a prediction result for the subject patient, based on the additional medical data and one or more features of the subject patient. In this case, the label information generated via the annotation operation may be used to train the machine learning model. Also, the machine learning model may include a weight associated with a plurality of nodes included in the machine learning model. In this case, the weight may include any parameter associated with the machine learning model.

In the disclosure, 'training' may refer to any process of changing a weight associated with the machine learning model by using training data and/or correct answer labels. According to an embodiment, training may refer to a process of changing or updating a weight associated with the machine learning model by performing forward propagation and backward propagation of the machine learning model (e.g., a first sub-prediction model) one or more times by using medical image data and correct answer labels (e.g., one or more features) of a subject to be learned. According to another embodiment, training may refer to a process of changing or updating a weight associated with the machine learning model by performing forward propagation and backward propagation of the machine learning model (e.g., a second sub-prediction model) one or more times by using one or more features extracted from medical image data of a subject to be learned and additional medical data and correct answer labels of the subject to be learned (e.g., a prognosis and/or a necessary intervention for the subject to be learned).

In the disclosure, 'label information' and/or 'correct answer labels' are correct answer information for a data sample and may refer to information obtained as a result of an annotation operation. The term correct answer label or correct answer may be interchangeably used with a term such as an annotation, annotation information, or a tag in the technical field. For example, the correct answer label, the correct answer, and the annotation information may refer to information determined according to an annotation operation performed by a user. As another example, the correct answer label, the correct answer, and the annotation information may refer to information determined according to an annotation operation performed by an annotation apparatus.

In the disclosure, 'each of a plurality of As' may refer to each of all components included in the plurality of As or may refer to each of some components included in the plurality of As. For example, each of a plurality of factors may refer to each of all factors included in the plurality of factors or may refer to each of some factors included in the plurality of factors.

In the disclosure, an 'instruction' is at least one instruction grouped based on a function and may refer to a component of a computer program, which is executed by a processor.

In the disclosure, a 'user' may refer to a person who uses a user terminal. For example, the user may include a medical staff, a patient, and a researcher who are provided with an interpretable prediction result for the patient. Also, a user may refer to a user terminal, and conversely, a user terminal may refer to a user. That is, the terms user and user terminal may be interchangeably used herein.

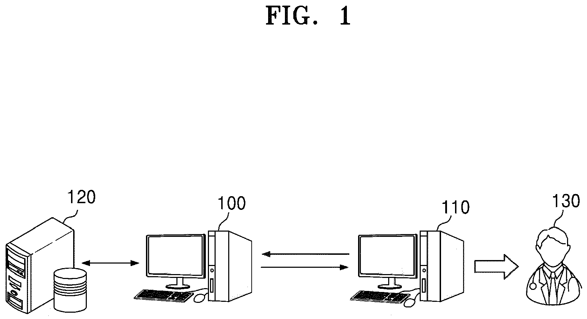

FIG. 1 illustrates a system in which an information processing system provides an interpretable prediction result for a patient, according to an embodiment of the disclosure. As shown in FIG. 1, a system for providing an interpretable prediction result for a patient may include an information processing system 100, a user terminal 110, and a storage system 120. In this case, the information processing system 100 may be connected to and configured to communicate with each of the user terminal 110 and the storage system 120. Although one user terminal 110 is illustrated in FIG. 1, the disclosure is not limited thereto. A plurality of user terminals 110 may be connected to and configured to communicate with the information processing system 100. Also, although the information processing system 100 is illustrated as one computing apparatus in FIG. 1, the disclosure is not limited thereto. The information processing system 100 may be configured to distribute and process information and/or data through a plurality of computing apparatuses. In addition, the storage system 120 is illustrated as one apparatus in FIG. 1, but the disclosure is not limited thereto. The storage system 120 may be configured as a plurality of storage apparatuses or a system supporting a cloud. Also, in FIG. 1, the components of the system for providing an interpretable prediction result for a patient represent functional elements that are functionally identifiable from each other, and a plurality of components may be implemented in a form in which the plurality of components are integrated with each other in an actual physical environment.

Each of the information processing system 100 and the user terminal 110 is any computing apparatus used to generate and provide an interpretable prediction result for a patient. In this case, the computing apparatus may refer to any type of apparatus having a computing function and may include, for example, a notebook computer, a laptop computer, a desktop computer, a server, or a cloud system, but is not limited thereto.

The information processing system 100 may receive medical image data of a subject patient and/or additional medical data of the subject patient. In this case, the additional medical data of the subject patient may include clinical data, lab data, and/or biological data of the subject patient. For example, the information processing system 100 may receive the medical image data of the subject patient and/or the additional medical data of the subject patient from the storage system 120 (e.g., an electronic medical record, a prescription delivery system, a medical image system, or a test information system) and/or the user terminal 110. The information processing system 100 may generate an interpretable prediction result for a patient and provide the same to a user 130 through the user terminal 110.

In an embodiment, the information processing system 100 may generate information about a prediction result for the subject patient, based on the medical image data of the subject patient and the additional medical data of the subject patient, by using a machine learning prediction model. In this case, the information about the prediction result for the subject patient may include information that may be used to treat the subject patient, and any information (clinical management) that may affect a treatment method for the subject patient. For example, such information may include a prognosis of the subject patient's condition, necessary interventions (e.g., a treatment plan and a treatment time) required for the patient in particular circumstances, drug activity, or changes in all clinically relevant values included in the body. Additionally or alternatively, the information processing system 100 may generate information about a factor affecting generation of the information about the prediction result for the subject patient, by using the machine learning prediction model.

The information processing system 100 may provide, to the user terminal 110, the information about the prediction result for the subject patient and/or the information about the factor affecting the generation of the information about the prediction result for the subject patient. The user terminal 110 may receive, from the information processing system 100, the information about the prediction result for the subject patient and/or the information about the factor, and may output the same through a display apparatus. That is, the user 130 (e.g., a medical staff, a patient, or a researcher) may make a medical intervention and/or a clinical determination for the subject patient, based on the information about the prediction result for the subject patient and/or the information about the factor.

The storage system 120 is an apparatus or a cloud system that stores and manages medical image data associated with the subject patient, additional medical data, and/or various types of data associated with a machine learning model, to provide an interpretable prediction result for the patient. For efficient management of data, the storage system 120 may store and manage various types of data by using a database. In this case, various types of data may include any data associated with the machine learning model and may include, for example, a file of target data, meta information about the target data, label information about the target data, which is a result of an annotation operation, data about the annotation operation, or a machine learning model (e.g., an artificial neural network model), but are not limited thereto. In FIG. 1, the information processing system 100 and the storage system 120 are illustrated as separate systems, but the disclosure is not limited thereto. The information processing system 100 and the storage system 120 may be integrated into one system.

According to some embodiments of the disclosure, the user 130 may take a necessary intervention for the subject patient and may receive assistance in making a clinical determination for the subject patient, by receiving information about a prediction result for a prognosis and/or a necessary intervention for the subject patient. Also, the user 130 may recognize a prediction reason for the prediction result for the subject patient by receiving information about factors affecting generation of the information about the prediction result, and accordingly, the necessary intervention for the subject patient and/or the clinical determination for the subject patient may be appropriately adjusted. Also, the information about the factors affecting the generation of the information about the prediction result may be used as new medical research data.

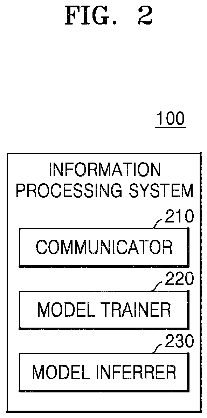

FIG. 2 is a block diagram illustrating an internal configuration of an information processing system, according to an embodiment of the disclosure. In order to generate an interpretable prediction result for a patient, the information processing system 100 may include a communicator 210, a model trainer 220, and a model inferrer 230 as shown in FIG. 2. In FIG. 2, components of the information processing system 100 represent functional elements that are functionally identifiable from each other, and a plurality of components may be implemented in a form in which the plurality of components are integrated into each other in an actual physical environment. Also, in FIG. 2, the information processing system 100 includes the communicator 210, the model trainer 220, and the model inferrer 230, but is not limited thereto. Some components may be omitted or other components may be added.

In an embodiment, the communicator 210 may receive medical image data of a subject patient and/or additional medical data of the subject patient. For example, the communicator 210 may receive medical image data of the subject patient and/or additional medical data of the subject patient from a medical imaging apparatus and/or a user terminal. Alternatively or additionally, the communicator 210 may receive medical image data and/or additional medical data of the subject patient from an internal and/or external storage apparatus of the information processing system 100. In another embodiment, the communicator 210 may receive training data for training the machine learning prediction model and provide the same to the model trainer 220. Also, in another embodiment, the communicator 210 may transmit pieces of information generated by the model inferrer 230 to the user terminal.

The model trainer 220 may generate and/or train (e.g., update) the machine learning prediction model. In this case, the machine learning prediction model may correspond to a model including a plurality of prediction models. For example, the machine learning prediction model may correspond to a 2-step stacking ensemble model including a first sub-prediction model and a second sub-prediction model. As another example, the machine learning prediction model may correspond to a model in which the first sub-prediction model and the second sub-prediction model are trained end-to-end. In an embodiment, the model trainer 220 may train the machine learning prediction model by using the training data received through the communicator 210. In this case, the training data may include medical image data and/or additional medical data of a subject to be learned. Additionally, the training data may include information (e.g., a doctor's clinical determination) about a prognosis and/or a necessary intervention for the subject to be learned. Additionally, the training data may include information about one or more features (e.g., phenotypic features) extracted from the medical image data of the subject to be learned.

The model trainer 220 may train the machine learning prediction model to generate reference information about a reference prediction result for a reference patient, based on medical image data of the reference patient and additional medical data of the reference patient, by using the training data described above. In an embodiment, the model trainer 220 may train the first sub-prediction model by using the medical image data of the subject to be learned and the information (i.e., correct answer labels) about the one or more features extracted from the medical image data of the subject to be learned. Also, the model trainer 220 may train the second sub-prediction model by using the information about the one or more features extracted from the medical image data of the subject to be learned, the additional medical data of the subject to be learned, and the information (i.e., correct answer labels) about the prognosis and/or the necessary intervention for the subject to be learned.

Accordingly, the first sub-prediction model may be trained to extract one or more reference features from the medical image data of the reference patient, and the second sub-prediction model may be trained to generate reference information about the reference prediction result for the reference patient, based on the additional medical data of the reference patient and the one or more reference features. In this case, the reference features may include interpretable reference phenotypic features that may be used to interpret information about the prediction result for the reference patient. The phenotypic features may include all types of phenomena comprehensible by a person within a medical image of the reference patient. Additionally, the model trainer 220 may train the second sub-prediction model to output reference information about the importance of each of a plurality of reference factors in generating the reference information about the reference prediction result for the reference patient. In this case, the plurality of reference factors may include one or more reference features and additional medical data of the reference patient, which are input to the second sub-prediction model. That is, the model trainer 220 may train the second sub-prediction model as an interpretable prediction model and may finally train the machine learning model as an interpretable prediction model.

The model inferrer 230 may generate information about a prediction result for the subject patient, based on the medical image data and the additional medical data of the subject patient, by using the machine learning prediction model. In this case, the information about the prediction result for the subject patient may include information about a prediction result for a prognosis and/or a necessary intervention for a subject patient. In an embodiment, the model inferrer 230 may extract one or more features from the medical image data of the subject patient by using the first sub-prediction model, and may generate information about the prediction result for the subject patient, based on the one or more features and the additional medical data of the subject patient, by using the second sub-prediction model.

The model inferrer 230 may generate information about a factor affecting generation of the information about the prediction result for the subject patient, by using the machine learning prediction model. In an embodiment, the model inferrer 230 may generate information about the importance of each of a plurality of factors in generating the information about the prediction result for the subject patient, by using the second sub-prediction model. In this case, the plurality of factors may include at least one of the additional medical data of the subject patient or the one or more features.

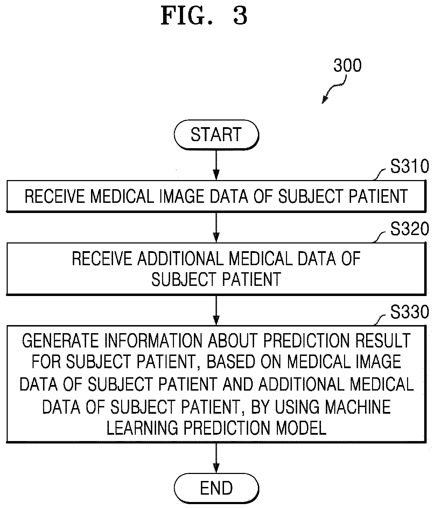

FIG. 3 is a flowchart illustrating a method of generating an interpretable prediction result for a patient, according to an embodiment of the disclosure. In an embodiment, a method 300 of generating an interpretable prediction result for a patient may be performed by a processor (e.g., at least one processor of an information processing system and/or at least one processor of a user terminal). The method 300 of generating an interpretable prediction result for a patient may be initiated when the processor receives medical image data of a subject patient (S310). Also, the processor may receive additional medical data of the subject patient (S320). FIG. 3 illustrates that the processor receives the medical image data of the subject patient in preference to the additional medical data, but is not limited thereto. For example, the processor may simultaneously receive the medical image data and the additional medical data of the subject patient. As another example, the processor may receive the additional medical data of the subject patient in preference to the medical image data.

The processor may generate information about a prediction result for the subject patient, based on the medical image data of the subject patient and the additional medical data of the subject patient, by using a machine learning prediction model (S330). For example, the processor may generate information about the prediction result for the subject patient based on data output in response to inputting the medical image data and the additional medical data of the subject patient to the machine learning prediction model. In this case, the machine learning prediction model may include a first sub-prediction model and a second sub-prediction model. In an embodiment of the disclosure, the processor may extract one or more features from the medical image data of the subject patient by using the first sub-prediction model, and may generate information about the prediction result for the subject patient, based on the one or more features and the additional medical data of the subject patient, by using the second sub-prediction model. For example, the processor may generate input data of the second sub-prediction model by concatenating the additional medical data of the subject patient with the one or more features, and may generate information about the prediction result for the subject patient by inputting the generated input data to the second sub-prediction model. In this case, the one or more features may include interpretable phenotypic features that may be used to interpret information about the prediction result for the subject patient.

In an embodiment, the processor may generate information about a factor affecting generation of the information about the prediction result for the subject patient, by using the machine learning prediction model. For example, the processor may obtain information about the importance of each of a plurality of factors in generating the information about the prediction result, by using the second sub-prediction model. In this case, the plurality of factors may include at least one of the additional medical data of the subject patient or the one or more features. Additionally or alternatively, the processor may determine at least one of the plurality of factors as a prediction reason, based on the information about the importance of each of the plurality of factors. Moreover, the processor may provide, to the user terminal, the information about the prediction result for the subject patient and/or the information about the factor.

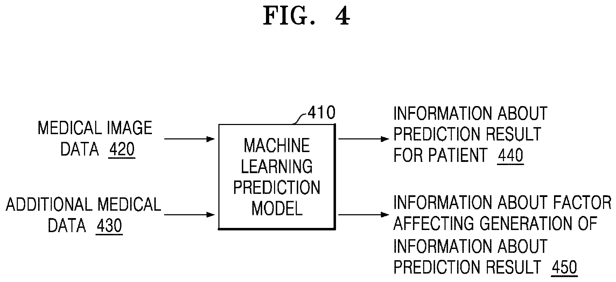

FIG. 4 illustrates an example of generating information about a prediction result for a patient and information about a factor affecting generation of the information about the prediction result by using a machine learning prediction model, according to an embodiment of the disclosure. In an embodiment, the processor may generate information 440 about a prediction result for a subject patient, by inputting medical image data 420 and additional medical data 430 of the subject patient to a machine learning prediction model 410. In this case, the machine learning prediction model 410 may correspond to a model trained to generate reference information about a reference prediction result for a reference patient, based on medical image data and additional medical data of the reference patient.

In order to simply perform prediction on the subject patient, a black-box type model (i.e., a model that outputs only prediction results without any prediction reason or ground) may be sufficient for the processor. However, in order for the processor to provide a reason or ground for prediction, an interpretable model may be required. For example, when a chest image (e.g., a chest radiographic image) is analyzed by using an existing general end-to-end deep learning model, though it may be predicted from the chest image that a patient needs mechanical ventilation, it may not be possible to infer a reason or ground for such prediction. That is, a user (e.g., a medical staff) who is provided with only results output from a deep learning model without any reason or ground for prediction may be unable to recognize why the patient needs mechanical ventilation.

As another example, even though the subject patient does not have any symptoms, such as fever and dyspnea, which are clinical grounds for a poor prognosis, a prediction result that the subject patient's prognosis is poor may be generated by the medical image data of the subject patient. In this case, in determining a clinical determination for the subject patient, it may be difficult for a medical staff to trust only prediction results without any grounds based on existing medical knowledge. In particular, when another subject patient's prognosis is predicted to be good even though the other subject patient has symptoms, such as fever and dyspnea, confusion may be caused in how a medical staff determines which patient to provide with limited resources first.

In order to solve the problems described above, the processor may generate information 450 (e.g., information about a factor that serves as a basis for generating information about a prediction result, information about a factor that is medically relevant for generating information about a prediction result, etc.) about a factor affecting generation of information about a prediction result, by inputting the medical image data 420 and the additional medical data 430 of the subject patient to the machine learning prediction model 410. In this case, the machine learning prediction model 410 may correspond to a model trained to generate reference information about a reference factor affecting generation of information about the reference prediction result. Accordingly, the processor may generate information about the prediction result for the subject patient and/or information about the factor by using the interpretable machine learning prediction model 410, and may provide the same to a user.

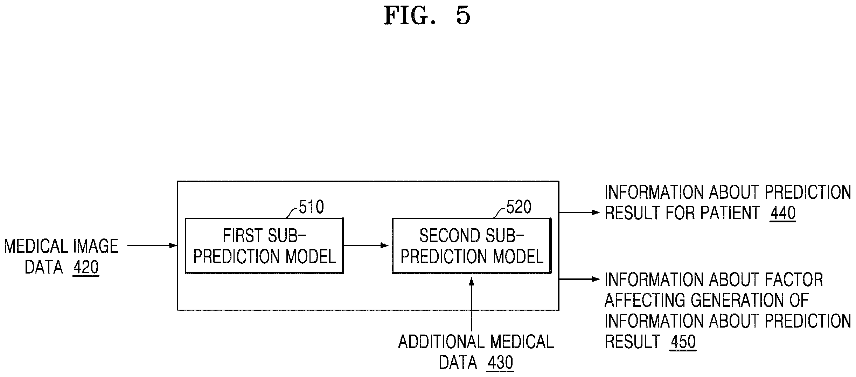

FIG. 5 illustrates an example of generating information about a prediction result for a patient and information about a factor affecting generation of the information about the prediction result by using a first sub-prediction model and a second sub-prediction model, according to an embodiment of the disclosure. Medical image data corresponds to complex data and thus may be suitable for a deep learning model, which is a black-box type model, and may be difficult to be applied to an interpretable model. Accordingly, when the processor performs prediction on a patient by using the medical image data, it may be difficult to provide a reason for such prediction. In order to solve such problems, the processor may use an interpretable machine learning prediction model, including a first sub-prediction model 510 and an interpretable second sub-prediction model 520, the first sub-prediction model 510 extracting one or more features from the medical image data 420, and the second sub-prediction model 520 generating a prediction result for the patient based on the one or more features. That is, the processor may first extract interpretable features from the medical image data 420 and perform prediction on the patient by using the extracted features. In this case, the one or more features extracted from the medical image data 420 may include interpretable phenotypic features that may be used to interpret the information about the prediction result for the patient. In the disclosure, the phenotypic features may include all types of phenomena comprehensible by a person within a medical image. For example, in the case of a lung image, phenotypic features may appear in the lungs, such as nodules, consolidation, pneumothorax, pleural effusion, cardiac hypertrophy, fibrosis, and mediastinal dilatation, and may include all feature points comprehensible by a person about the corresponding part. As another example, in the case of an ultrasound image of the thyroid gland, cystic change, microcalcification, increased blood flow in the periphery, high echogenicity, loss of fatty hilum, etc. may be extracted as phenotypic features.

In this case, the first sub-prediction model 510 included in the machine learning prediction model may correspond to a deep learning model (e.g., an interpretable phenotype extractor) that extracts interpretable features (e.g., phenotypic features) from complex medical image data 420. Also, the second sub-prediction model 520 included in the machine learning prediction model may correspond to an interpretable model (e.g., a prognosis prediction model), which predicts a prognosis and/or a necessary intervention for a patient based on the interpretable features extracted by the first sub-prediction model 510. The machine learning prediction model includes the first sub-prediction model 510 and the second sub-prediction model 520, and thus may finally correspond to an interpretable prediction model.

In an embodiment, the processor may extract one or more features from the medical image data 420 of the subject patient, by using the first sub-prediction model 510. Then, the processor may generate the information 440 about the prediction result for the subject patient, based on the one or more features and the additional medical data 430 of the subject patient, by using the second sub-prediction model 520. For example, the processor may generate input data of the second sub-prediction model 520 by concatenating the one or more features with the additional medical data 430 of the subject patient, and may generate the information 440 about the prediction result for the subject patient by inputting the generated input data to the second sub-prediction model 520. In this case, the first sub-prediction model 510 may be trained to extract one or more reference features from medical image data of a reference patient, and the second sub-prediction model 520 may be trained to generate reference information about a reference prediction result for the reference patient based on the one or more reference features and additional medical data of the reference patient.

The processor may generate information 450 (e.g., the importance of each factor, an importance ranking, etc.) about a factor affecting generation of the information 440 about the prediction result for the subject patient, by using the machine learning prediction model. In an embodiment, the processor may generate the information 450 about the factor affecting the generation of the information about the prediction result, based on the one or more features and the additional medical data 430 of the subject patient, by using the second sub-prediction model 520. For example, the processor may obtain information about the importance of each of a plurality of factors in generating the information 440 about the prediction result for the subject patient, by using the second sub-prediction model 520. In this regard, the plurality of factors may include the additional medical data 430 of the subject patient and/or the one or more features. In this case, the second sub-prediction model 520 may correspond to a model trained to calculate a numerical value indicating a degree of impact of each of the plurality of factors as the importance of each of the plurality of factors in performing prediction on the subject patient (i.e., in generating information about a prediction result for the subject patient). Additionally or alternatively, the processor may determine at least one of the plurality of factors as a prediction reason, based on the information about the importance of each of the plurality of factors.

For example, when the processor generates information about a prediction result that the subject patient needs mechanical ventilation, by using the machine learning prediction model, a numeral value of a factor called consolidation, which is a phenotypic feature extracted from the medical image data of the subject patient, may have a significant impact. In this case, the processor may determine the subject patient's symptom of consolidation as a prediction reason or ground for mechanical ventilation. The processor may allow a user (e.g., a medical staff) to recognize that the subject patient needs mechanical ventilation due to the subject patient's symptom of consolidation, by providing the determined prediction reason to the user terminal.

As shown in FIG. 5, pieces of final output data of the first sub-prediction model 510 may be used as input data of the second sub-prediction model 520. Additionally or alternatively, features output from a hidden layer before a final output layer of the first sub-prediction model 510 may be used as input data of the second sub-prediction model 520. In this case, the features output from the hidden layer may include two or higher-dimensional data extracted from a convolutional layer and/or data (e.g., values) extracted from a layer, such as a fully connected layer. Also, in FIG. 5, the additional medical data 430 is input to the second sub-prediction model 520, but the disclosure is not limited thereto. For example, the additional medical data 430 may be input to at least one of layers included in the first sub-prediction model 510.

FIG. 5 illustrates an example of a prediction model including the first sub-prediction model 510 and the second sub-prediction model 520, but is not limited thereto. For example, the machine learning prediction model may correspond to a 2-step stacking ensemble model. As another example, the machine learning prediction model may correspond to a model in which a first sub-prediction model and a second sub-prediction model are trained end-to-end by using a deep learning framework (e.g., Tensorflow, PyTorch, etc.).

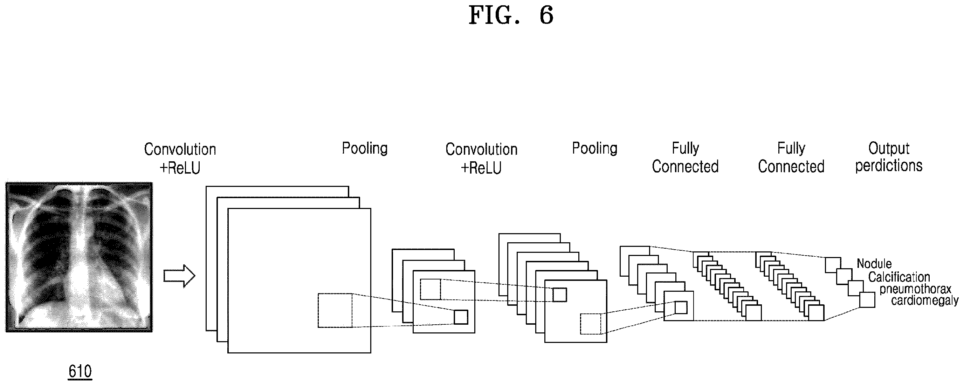

FIG. 6 illustrates an example of extracting one or more features from medical image data by using a convolutional neural network (CNN)-based first sub-prediction model, according to an embodiment of the disclosure. The processor may extract one or more features from medical image data by using the first sub-prediction model. The one or more features extracted from the medical image data may include interpretable phenotypic features that may be used to interpret information about a prediction result for a patient. In this case, the phenotypic features may include information that may be interpreted from medical image data in diagnosing the patient. For example, the phenotypic features may include information regarding expression of an abnormality, such as pneumothorax, mediastinal widening, pneumoperitoneum, nodule/mass, consolidation, pleural effusion, linear atelectasis, fibrosis, calcification, cardiomegaly, or tuberculosis, but it not limited thereto. As another example, the phenotypic features may include interpretable information, such as a shape, size, and location of the lungs, as well as a medical abnormality.

In an embodiment, the processor may extract one or more features from the medical image data by using the CNN-based first sub-prediction model (e.g., a CNN-based interpretable phenotype extractor). In this case, a CNN algorithm, which serves as a basis of the first sub-prediction model, may process not only images but also multi-dimensional complex data, such as three or higher-dimensional data. As shown in FIG. 6, the CNN algorithm may increase the number of pieces of data and reduce the size of multi-dimensional data through convolution and pooling, and may finally vectorize and process data as a one-dimensional fully connected layer. The CNN algorithm may be used as a tool to perform feature selection and may also be used as a tool to hide sensitive personal information that are recognizable by a person in an image.

The CNN algorithm may be modified into various structures, and when the CNN algorithm is trained with data and correct answers (labels) suitable for a purpose, a weight may be modified by searching for particular patterns (e.g., phenotypic features) in an image (e.g., medical image data). In this case, the correct answers may include one or more features interpretable from the medical image data. For example, the first sub-prediction model may be trained to predict a numerical value of each phenotypic feature based on training medical image data and information (i.e., correct answer labels and annotation information) about various phenotypic features in the training medical image data.

As shown in FIG. 6, the processor may extract one or more phenotypic features (e.g., information, data, and numerical values about nodules, calcification, pneumothorax, cardiomegaly, etc.) based on output data, by inputting medical image data 610 (e.g., a chest image) of a subject patient to the CNN-based first sub-prediction model. FIG. 6 illustrates that X-ray data of the chest image of the subject patient is input to the first sub-prediction model, but is not limited thereto. For example, different types of medical image data, such as MRI and CT, may be input.

Also, the first sub-prediction model is not limited to a CNN structure shown in FIG. 6 and may correspond to any type of CNN-based structure. For example, the CNN structure including an input layer and an output layer may include EfficientNet, Xception, VGG, ResNet, Inception, MobileNet, DenseNet, NASNet, etc. of all sizes.

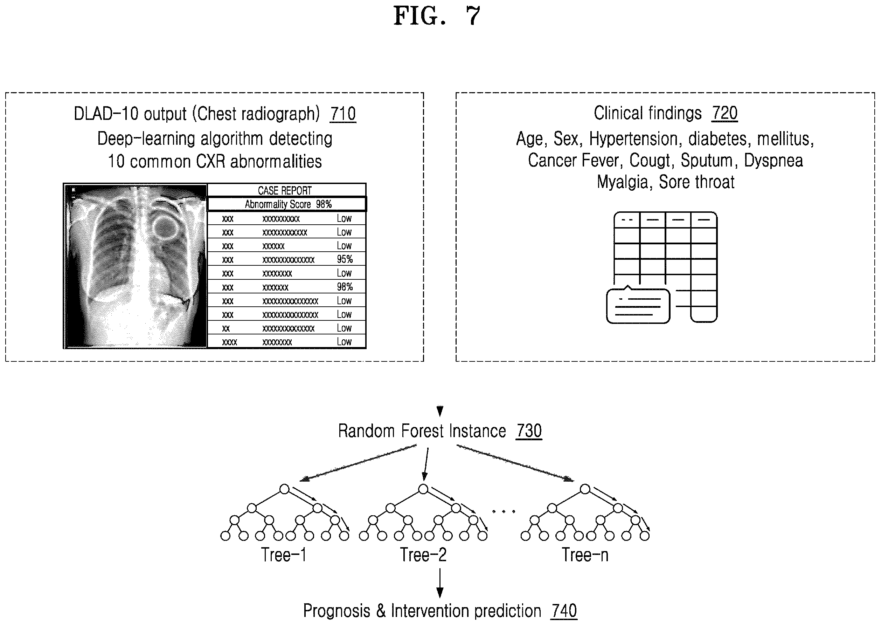

FIG. 7 illustrates an example of generating information about a prediction result for a patient by using a second sub-prediction model, according to an embodiment of the disclosure. The processor may generate information about a prediction result for a subject patient, based on one or more features extracted from additional medical data and medical image data of the subject patient, by using the second sub-prediction model. In an embodiment, the processor may predict a prognosis and/or a necessary intervention for the subject patient, based on interpretable phenotypic features extracted from the medical image data and additional medical data of the subject patient. For example, the processor may generate information about a prediction result of whether mechanical ventilation is required, based on the phenotypic features extracted from a chest image of the subject patient and additional medical data of the subject patient, by using the second sub-prediction model.

To this end, the second sub-prediction model may be trained to predict a prognosis and/or a necessary intervention for the patient. Training data of the second sub-prediction model may include phenotypic features extracted from medical image data of a subject to be learned and additional medical data of the subject to be learned. Also, the training data of the second sub-prediction model may include, as correct answer labels, information about the prognosis and/or the necessary intervention for the subject to be learned. In this case, the information about the prognosis of the subject to be learned may include information about occurrence of a new event of the subject to be learned, such as death, intensive care unit (ICU) admission, recurrence of disease, metastasis of cancer, or cardiac arrest, and/or information about a time when an event occurred from a particular reference time (e.g., a time when an image of training data is captured and/or a time when training data is obtained).

As shown in FIG. 7, the processor may generate information 740 ('Prognosis & Intervention prediction') about a prediction result for a prognosis and/or a necessary intervention for a subject patient, by inputting a phenotypic feature 710 ('DLAD-10 output') extracted from a chest image of the subject patient and additional medical data 720 ('Clinical finding') of the subject patient to a second sub-prediction model 730 based on random forest instance. For example, the phenotypic feature 710 extracted from the chest image of the subject patient may include information about calcification, atelectasis, fibrosis, pneumothorax, cardiomegaly, nodule, pleural effusion, pneumoperitoneum, mediastinal widening, consolidation, etc. Also, the additional medical data 720 ('Clinical finding') may include clinical data (e.g., fever, sputum, age, sex, dyspnea, hypertension, diabetes, cancer, or underlying disease) and/or lab data (e.g., neutrophil, lymphocyte, platelet, CRP, LDH, D-dimer, SP02, Ct:E, or RdRP) of the subject patient.

In this case, the information about the prediction result for the subject patient's prognosis may include information about prediction of occurrence of a new event of the subject patient, such as death, survival, ICU admission, disease recurrence, cancer metastasis, or cardiac arrest, the likelihood of occurrence of an event, a time when an event is predicted to occur, etc. For example, the information about the prediction result for the subject patient's prognosis may include information about a classification result for predicting an event. Additionally or alternatively, the information about the prediction result for the subject patient's prognosis may include a result of a censored problem that predicts a particular event and a time of occurrence. Also, the prediction result for the necessary intervention for the subject patient may include information indicating whether a medical activity (or resource), such as oxygen supplementation, mechanical ventilation, extracorporeal membrane oxygenation (ECMO), medicine, or surgery, is required.

In FIG. 7, a random forest instance-based model is illustrated as an example of the second sub-prediction model, but the disclosure is not limited thereto. For example, in the case of a binary classification problem, a back-bone model of the second sub-prediction model may correspond to a prediction model, such as interpretable deep neural network (iDNN), random forest, SVM, boost, bagging, Lasso, Ridge, or ElasticNet, but is not limited thereto. As another example, in the case of survival prediction as censored data, the second sub-prediction model may correspond to a survival prediction model, such as random forest survival regression classification (RFSRC), a regression model (e.g., cox proportional hazard model, Lasso, Ridge, or ElasticNet) with C-index or Coxph as a family, or XGBoost for survival analysis (XGBSE).

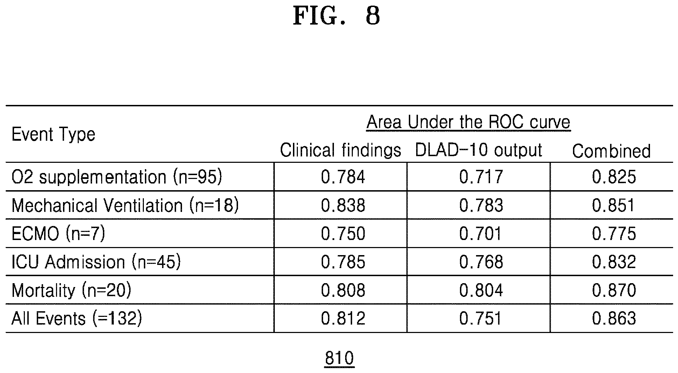

FIG. 8 is a table illustrating prediction performance when prediction is performed on a patient, according to an embodiment of the disclosure. In Table 810 shown in FIG. 8, when prediction is performed on each event type, such as oxygen (O2) supplementation, mechanical ventilation, ECMO, ICU admission, mortality, and all events, 'Area under the ROC curve' values of a case ('Clinical findings') where prediction is performed using only additional medical data, a case ('DLAD-10 output') where prediction is performed using only medical image data, and a case ('Combined') where prediction is performed using both medical image data and additional medical data may be represented. In this regard, the case where prediction is performed using only medical image data may correspond to a case where prediction is performed using 10 phenotypic features (e.g., calcification, atelectasis, fibrosis, pneumothorax, cardiomegaly, Nodule, pleural effusion, pneumoperitoneum, mediastinal widening, and consolidation) extracted from the medical image data. As shown in FIG. 8, in the case ('Combined') where prediction is performed using both medical image data and additional medical data, the performance may be improved compared to the case ('Clinical findings' or 'DLAD-10 output') where prediction is performed using only medical image data or additional medical data.

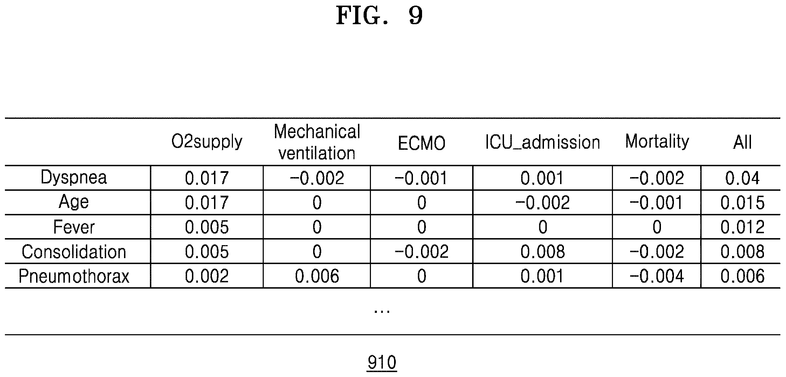

FIG. 9 is a table illustrating impacts of a plurality of factors in performing prediction on a subject patient, according to an embodiment of the disclosure. In an embodiment, the processor may generate information (e.g., information about a factor related to generation of information about a prediction result for a subject patient) about a factor affecting generation of information about a prediction result for the subject patient, by using a machine learning prediction model. For example, the processor may obtain information about the importance of each of a plurality of factors in generating the information about the prediction result for the subject patient, by using a second sub-prediction model. Additionally, the processor may determine at least one of the plurality of factors as a prediction reason, based on the information about the importance of each of the plurality of factors. In this regard, the plurality of factors may include additional medical data of the subject patient and/or one or more features.

In Table 910 shown in FIG. 9, when prediction is performed on a subject patient's prognosis and/or each of necessary interventions ('02 supplementation (O2supply)', 'Mechanical ventilation', 'ECMO', 'ICU admission', 'Mortality', and 'All'), an impact of each of a plurality of factors ('Dyspnea', 'Age', 'Fever', 'Consolidation', and 'Pneumothorax') is represented in a numerical value. For example, when the numerical value is a positive number, a higher value of a corresponding factor may indicate that a corresponding intervention is required. That is, a higher positive numerical value may indicate a greater impact (i.e., a higher importance) of the corresponding factor in generating information about a corresponding prediction result. As another example, when the numerical value is a negative value, a higher value of the corresponding factor may indicate that the corresponding intervention is not required.

As shown in Table 910, as with previously known medical knowledge, factors 'Dyspnea', 'Age', and 'Fever' may have a significant impact on prediction of the patient's prognosis and/or some (e.g., 'O2 supply') of the necessary interventions. Among the plurality of factors in Table 910, 'Consolidation' and 'Pneumothorax' may correspond to interpretable phenotypic features extracted from a chest image of the subject patient. When prediction is performed on the subject patient with an absolute value of each pixel of the chest image by using only an existing deep learning model, a user may be able to receive information that the subject patient needs O2 supplementation, but may be unable to receive information about phenotypic features, such as consolidation and pneumothorax, and information about why the subject patient needs O2 supplementation. That is, a user (e.g., a medical staff) may be unable to recognize which part of the chest image has affected a prediction result for a prognosis and/or a necessary intervention for the subject patient and may be unable to use information about phenotypic features extracted from the chest image.

In contrast, according to some embodiments of the disclosure, the processor may extract, and provide, phenotypic features comprehensible by a medical staff from medical image data of the subject patient, and may provide phenotypic features affecting prediction on the subject patient. For example, the processor may provide, to the user, information that the subject patient has a poor prognosis due to the presence of consolidation in the lungs and the subject patient needs O2 supplementation. The user (e.g., the medical staff) may make a clinical determination based on the information provided as described above that consolidation is common in pneumonia and O2 supplementation is required due to a reduced gas exchange surface caused by an increase in inflammatory substances.

According to an embodiment, when it is predicted by the machine learning prediction model that a first patient has no clinical symptoms but has a poor prognosis and a second patient has clinical symptoms but has a good prognosis, the user may know that such a prediction result is due to consolidation extracted from the medical image data of the subject patient, and may trust the prediction result. Accordingly, the user may determine to use limited resources for the first patient, who is predicted to have a poor prognosis despite no clinical symptoms, between the first patient and the second patient.

According to some embodiments of the disclosure, based on pieces of information provided, the user may recognize what clinically important factors (e.g., phenotypic features) are, and may be helped in determining a patient's treatment plan. Also, based on the pieces of information provided, the user may consider a new hypothesis (e.g., a medical research hypothesis) or a treatment plan. For example, when the use of ECMO is predicted to be important in a patient with fibrosis, in the future, an intervention may be taken by providing ECMO to the patient with fibrosis early, so that the patient's prognosis may be quickly improved. As another example, when the use of ECMO is predicted to be important in a patient with fibrosis and the number of ECMO machines is limited, ECMO machines are prepared in advance according to the number of patients with symptoms of fibrosis, such that the user may efficiently manage resources. Also, as another example, what is identified as a factor for the use of ECMO in patients with fibrosis may be provided as an important role in initiating new research into why ECMO is required and effective in patients with fibrosis.

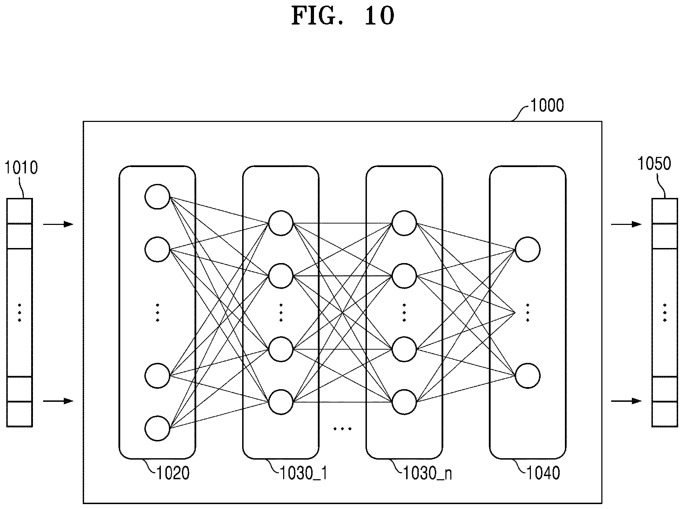

FIG. 10 is an exemplary diagram illustrating an artificial neural network model according to an embodiment of the disclosure. An artificial neural network model 1000 is an example of a machine learning model, and in machine learning technology and cognitive science, is a statistical learning algorithm implemented based on the structure of a biological neural network, or a structure for executing the algorithm.

According to an embodiment, the artificial neural network model 1000 is trained so that an error between a correct output corresponding to a particular input and an inferred output are reduced, as nodes repeatedly adjust a weight of a synapse as in the biological neural network, the nodes being artificial neurons forming a network by combining synapses, and thus, the artificial neural network model 1000 may represent a machine learning model with problem-solving ability. For example, the artificial neural network model 1000 may include arbitrary probabilistic models, neural network models, etc. used in artificial intelligence learning methods, such as machine learning and deep learning.

According to an embodiment, the artificial neural network model 1000 may include an artificial neural network model configured to perform prediction of at least one of a prognosis or a necessary intervention for a subject patient (e.g., to generate information about a prediction result), based on input medical image data and additional medical data of the subject patient. Additionally or alternatively, the artificial neural network model 1000 may include an artificial neural network model configured to extract one or more features from input medical image data of the subject patient. Additionally or alternatively, the artificial neural network model 1000 may include an artificial neural network model configured to perform prediction of at least one of a prognosis or a necessary intervention for the subject patient, based on one or more input features and input additional medical data of the subject patient.

The artificial neural network model 1000 may be implemented as a multilayer perceptron (MLP) including multilayer nodes and connections therebetween. According to the present embodiment, the artificial neural network model 1000 may be implemented as one of various artificial neural network model structures each including an MLP. As shown in FIG. 10, the artificial neural network model 1000 may include an input layer 1020 which receives an input signal or data 1010 from the outside, an output layer 1040 which outputs an output signal or data 1050 corresponding to the input data, and n hidden layers 1030_1 to 1030_n which are positioned between the input layer 1020 and the output layer 1040 and are configured to receive signals from the input layer 1020, extract characteristics therefrom, and transmit the characteristics to the output layer 1040. In this case, the output layer 1040 receives signals from the hidden layers 1030_1 to 1030_n and outputs the signals to the outside.

Learning methods of the artificial neural network model 1000 may include a supervised learning method that performs learning to be optimized to solve a problem by an input of a teacher signal (a correct answer), and an unsupervised learning method that does not require a teacher signal. In an embodiment, the information processing system may perform supervised learning and/or unsupervised learning of the artificial neural network model 1000 to generate information about a prediction result for a subject patient based on medical image data and additional medical data of the subject patient. For example, the information processing system may perform supervised learning of the artificial neural network model 1000 to generate reference information about a reference prediction result for a reference patient based on medical image data and additional medical data of the reference patient.

In another embodiment, the information processing system may perform supervised learning and/or unsupervised learning of the artificial neural network model 1000 to extract one or more features from medical image data of the subject patient. For example, the information processing system may perform supervised learning of the artificial neural network model 1000 to extract one or more reference features (e.g., phenotypic features) from reference medical image data of the reference patient. Also, in another embodiment, the information processing system may perform supervised learning and/or unsupervised learning of the artificial neural network model 1000 to generate information about a prediction result for the subject patient based on one or more features extracted from medical image data of the subject patient, and additional medical data of the subject patient. For example, the information processing system may perform supervised learning of the artificial neural network model 1000 to generate reference information about a reference prediction result for a reference patient based on reference features extracted from medical image data of the reference patient, and additional medical data of the reference patient.

The artificial neural network model 1000 trained as described above may be stored in a memory (not shown) of the information processing system, and may generate a prediction result for the subject patient by performing prediction on the subject patient, in response to an input of the medical image data and the additional medical data of the subject patient, which are received from a communication module and/or the memory. Additionally or alternatively, the artificial neural network model 1000 may extract one or more features in response to an input of medical image data of the subject patient. Additionally or alternatively, the artificial neural network model 1000 may generate a prediction result for the subject patient, by performing prediction on the subject patient, in response to one or more features extracted from medical image data of the subject patient, and additional medical data of the subject patient.

According to an embodiment, an input variable of an artificial neural network model that generates information about the prediction result for the subject patient may include medical image data and/or additional medical data of the subject patient. For example, an input variable input to the input layer 1020 of the artificial neural network model 1000 may include an image vector 1010 including medical image data of the subject patient as one vector data element, and/or a vector 1010 including additional medical data of the subject patient as one vector data element. In response to the input, an output variable output from the output layer 1040 of the artificial neural network model 1000 may include a vector 1050 indicating or characterizing information about the prediction result for the subject patient. That is, the output layer 1040 of the artificial neural network model 1000 may be configured to output a vector indicating or characterizing information about a prediction result for the subject patient. In the disclosure, the output variable of the artificial neural network model 1000 is not limited to the types described above, and may include any information/data indicating information about the prediction result for the subject patient. In addition, the output layer 1040 of the artificial neural network model 1000 may be configured to output a vector indicating reliability and/or accuracy, such as information about the prediction result for the subject patient. Additionally or alternatively, the output layer 1040 of the artificial neural network model 1000 may be configured to output a vector indicating information about a factor affecting generation of information about the prediction result for the subject patient.

In another embodiment, an input variable of a machine learning model which extracts one or more features from medical image data, that is, the artificial neural network model 1000, may include medical image data of the subject patient. For example, an input variable input to the input layer 1020 of the artificial neural network model 1000 may include an image vector 1010 including medical image data of the subject patient as one vector data element. In response to the input of the medical image data of the subject patient, an output variable output from the output layer 1040 of the artificial neural network model 1000 may include a vector 1050 indicating or characterizing one or more features (e.g., phenotypic features). In the disclosure, the output variable of the artificial neural network model 1000 is not limited to the types described above, and may include any information/data indicating one or more features.

Also, according to another embodiment, an input variable of an artificial neural network model that generates information about the prediction result for the subject patient may include one or more features extracted from medical image data of the subject patient, and/or additional medical data of the subject patient. For example, an input variable input to the input layer 1020 of the artificial neural network model 1000 may include a vector 1010 including one or more features and/or additional medical data of the subject patient as one vector data element. In response to the input, an output variable output from the output layer 1040 of the artificial neural network model 1000 may include a vector 1050 indicating or characterizing information about the prediction result for the subject patient. That is, the output layer 1040 of the artificial neural network model 1000 may be configured to output a vector indicating or characterizing information about the prediction result for the subject patient. In the disclosure, the output variable of the artificial neural network model 1000 is not limited to the types described above, and may include any information/data indicating information about the prediction result for the subject patient. In addition, the output layer 1040 of the artificial neural network model 1000 may be configured to output a vector indicating reliability and/or accuracy, such as information about the prediction result for the subject patient. Additionally or alternatively, the output layer 1040 of the artificial neural network model 1000 may be configured to output a vector indicating information about a factor affecting generation of information about the prediction result for the subject patient.

As described above, a plurality of input variables and a plurality of output variables corresponding to the plurality of input variables are respectively matched to the input layer 1020 and the output layer 1040 of the artificial neural network model 1000, and a synaptic value between nodes included in the input layer 1020, the hidden layers 1030_1 to 1030_n, and the output layer 1040, is adjusted, such that training may be performed to extract a correct output corresponding to a particular input. Through such a training process, characteristics hidden in input variables of the artificial neural network model 1000 may be identified, and a synaptic value (or weight) between nodes of the artificial neural network model 1000 may be adjusted so that an error between an output variable calculated based on an input variable and a target output is reduced. The artificial neural network model 1000 trained as described above may output information about the prediction result for the subject patient, in response to the input of medical image data and additional medical data of the subject patient. Additionally or alternatively, the artificial neural network model 1000 trained as described above may output one or more features in response to the input of medical image data of the subject patient. Additionally or alternatively, the artificial neural network model 1000 trained as described above may output information about the prediction result for the subject patient, in response to the input of one or more features extracted from medical image data of the subject patient, and additional medical data of the subject patient.

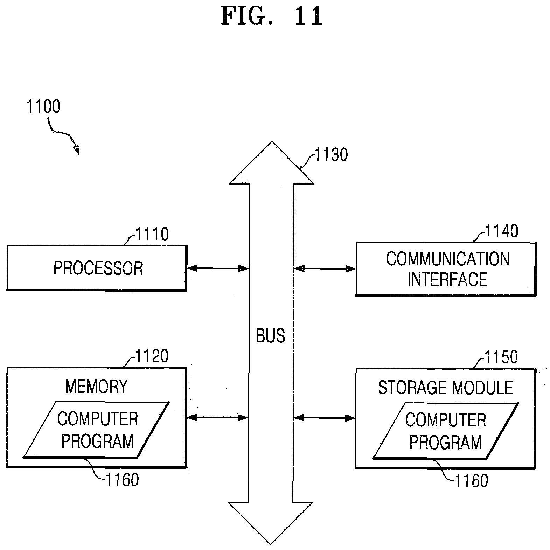

FIG. 11 is a block diagram of a computing apparatus (e.g., an information processing system) for generating an interpretable prediction result for a patient, according to an embodiment of the disclosure. As shown in FIG. 11, a computing apparatus 1100 may include one or more processors 1110, a bus 1130, a communication interface 1140, a memory 1120 which loads a computer program 1160 executed by the processors 1110, and a storage module 1150 which stores a computer program 1160. However, only components related to the embodiment of the disclosure are shown in FIG. 11. Accordingly, those of ordinary skill in the art will understand that the disclosure may further include general-purpose components other than those shown in FIG. 11.

The processors 1110 controls the overall operation of each component of the computing apparatus 1100. The processors 1110 may include a CPU, a microprocessor unit (MPU), a micro controller unit (MCU), a graphic processing unit (GPU), or any type of processor well known in the art of the disclosure. Also, the processors 1110 may perform an operation on at least one application or program for executing the methods according to the embodiments of the disclosure. The computing apparatus 1100 may include one or more processors.

The memory 1120 may store various types of data, commands, and/or information. The memory 1120 may load one or more computer programs 1160 from the storage module 1150 to execute the methods/operations according to various embodiments of the disclosure. The memory 1120 may be implemented as a volatile memory, such as RAM, but the technical scope of the disclosure is not limited thereto.

The bus 1130 may provide a communication function between the components of the computing apparatus 1100. The bus 1130 may be implemented as various types of buses, such as an address bus, a data bus, and a control bus.

The communication interface 1140 may support wired/wireless Internet communication of the computing apparatus 1100. Also, the communication interface 1140 may support various communication methods other than Internet communication. To this ends, the communication interface 1140 may include a communication module well known in the art of the disclosure.

The storage module 1150 may non-temporarily store the one or more computer programs 1160. The storage module 1150 may include a nonvolatile memory, such as ROM, EPROM, EEPROM, and a flash memory, a hard disk, a removable disk, or any type of computer-readable recording medium well known in the art to which the disclosure pertains.

The computer program 1160 may include one or more instructions that, when loaded into the memory 1120, cause the processors 1110 to perform the operations/methods according to various embodiments of the disclosure. That is, the processors 1110 may perform the operations/methods according to various embodiments of the disclosure by executing the one or more instructions.

For example, the computer program 1160 may include one or more instructions to perform operations of receiving medical image data of a subject patient, receiving additional medical data of the subject patient, and generating information about a prediction result for the subject patient based on the medical image data of the subject patient and the additional medical data of the subject patient, by using a machine learning prediction model. In this case, a system for generating an interpretable prediction result for a patient according to some embodiments of the disclosure may be implemented by the computing apparatus 1100.

The above descriptions of the disclosure are provided to enable those of ordinary skill in the art to execute or use the disclosure. Various modifications of the disclosure will be readily apparent to those of ordinary skill in the art, and the generic principles defined herein may be applied to various modifications without departing from the spirit or scope of the disclosure. Accordingly, the disclosure is not intended to be limited to the examples set forth herein, but is to be accorded the widest scope consistent with the principles and novel features disclosed herein.

Although example implementations may refer to utilizing aspects of the presently disclosed subject matter in the context of one or more standalone computer systems, the subject matter is not so limited, rather, it may be implemented in connection with any computing environment, such as a network or distributed computing environment. Further, aspects of the presently disclosed subject matter may be implemented in or across a plurality of processing chips or apparatuses, and a storage may be similarly affected across the plurality of apparatuses. Such apparatuses may include personal computers (PCs), network servers, and handheld apparatuses.

Although the disclosure has been described herein in connection with some embodiments, it should be understood that various modifications and changes can be made without departing from the scope of the disclosure that can be understood by those skilled in the art to which the disclosure pertains. In addition, such modifications and changes are intended to fall within the scope of the claims appended hereto.

According to some embodiments of the disclosure, in order to generate an interpretable prediction result for a patient, a machine learning model (i.e., a multimodal-based model) that receives not only image data but also various types of data as inputs may be used. Accordingly, a subject patient's prognosis may be predicted by using additional medical data (e.g., clinical data, biological data, lab data, etc.) as well as medical image data of the subject patient, and the accuracy of prediction may be improved.

According to some embodiments of the disclosure, by receiving information about prediction results for prognoses and/or necessary interventions for patients, a user (e.g., a medical staff) may efficiently and effectively manage limited resources (e.g., personnel, apparatuses, and drugs). For example, when there is an outbreak of an infectious disease and many patients need to be taken care of in an emergency with limited medical resources, medical resources are preferentially provided to emergency patients or patients with potential for improvement in prognosis according to information about prediction results for patients, such that prognoses of many patients may be improved.

According to some embodiments of the disclosure, an interpretable machine learning prediction model may be provided to perform interpretable prediction on a patient. In this case, the interpretable machine learning prediction model may include a model that extracts pieces of information (e.g., phenotypic features) interpretable by a user from medical image data, and a model that provides information (e.g., an importance ranking) about the importance of each of a plurality of factors in performing prediction. Accordingly, the user may be provided with information about a prediction result for the patient and/or information about a factor affecting prediction on the patient. That is, the user may further trust information about the prediction result based on information about the factor and may make a clinical determination with appropriate grounds based on pieces of information provided.

The effects of the disclosure are not limited to the aforementioned effects, and other effects not mentioned may be clearly understood by those of ordinary skill in the art to which the disclosure pertains (hereinafter, referred to as 'those of ordinary skill in the art') from the description of the claims.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A method, performed by at least one computing apparatus, of generating an interpretable prediction result for a patient, the method comprising:
receiving medical image data of a subject patient;
receiving additional medical data of the subject patient; and
generating information about a prediction result for the subject patient, based on the medical image data of the subject patient and the additional medical data of the subject patient, by using a machine learning prediction model.

2. The method of claim 1, further comprising
generating information about a factor affecting generation of the information about the prediction result for the subject patient, by using the machine learning prediction model.

3. The method of claim 2, further comprising
providing, to a user terminal, at least one of the information about the prediction result for the subject patient or the information about the factor.

4. The method of claim 1, wherein
the machine learning prediction model comprises a first sub-prediction model and a second sub-prediction model, and
the generating of the information about the prediction result for the subject patient comprises:
extracting one or more features from the medical image data of the subject patient, by using the first sub-prediction model; and
generating the information about the prediction result for the subject patient, based on the one or more features and the additional medical data of the subject patient, by using the second sub-prediction model.

5. The method of claim 4, further comprising
generating information about a factor affecting generation of the information about the prediction result for the subject patient, by using the machine learning prediction model,
wherein the generating of the information about the factor comprises
obtaining information about an importance of each of a plurality of factors in generating the information about the prediction result for the subject patient, by using the second sub-prediction model,
wherein the plurality of factors comprise at least one of the additional medical data of the subject patient or the one or more features.

6. The method of claim 5, wherein
the generating of the information about the factor further comprises
determining at least one of the plurality of factors as a prediction reason, based on the information about the importance.

7. The method of claim 4, wherein
the one or more features comprise a phenotypic feature that is usable to interpret the information about the prediction result for the subject patient.

8. The method of claim 4, wherein
the first sub-prediction model is trained to extract one or more reference features from medical image data of a reference patient, and
the second sub-prediction model is trained to generate reference information about a reference prediction result for the reference patient, based on additional medical data of the reference patient and the one or more reference features.

9. The method of claim 4, wherein
the generating of the information about the prediction result for the subject patient, based on the one or more features and the additional medical data of the subject patient, by using the second sub-prediction model comprises:
generating input data of the second sub-prediction model by concatenating the additional medical data of the subject patient with the one or more features; and
generating the information about the prediction result for the subject patient by inputting the generated input data to the second sub-prediction model.

10. The information processing system of claim 1, wherein
the additional medical data of the subject patient comprises at least one of clinical data, lab data, or biological data of the subject patient.

11. A computer program stored in a computer-readable recording medium for executing, on a computer, the method of generating the interpretable prediction result for the patient according to any one of claims 1 to 10.

12. An information processing system comprising:
a memory storing one or more instructions; and
a processor configured to execute the one or more stored instructions to
receive medical image data of a subject patient,
receive additional medical data of the subject patient, and
generate information about a prediction result for the subject patient, based on the medical image data of the subject patient and the additional medical data of the subject patient, by using a machine learning prediction model.

13. The information processing system of claim 12, wherein
the processor is further configured to
generate information about a factor affecting generation of the information about the prediction result for the subject patient, by using the machine learning prediction model.

14. The information processing system of claim 13, wherein
the processor is further configured to
provide, to a user terminal, at least one of the information about the prediction result for the subject patient or the information about the factor.

15. The information processing system of claim 12, wherein
the machine learning prediction model comprises a first sub-prediction model and a second sub-prediction model, and
the processor is further configured to
extract one or more features from the medical image data of the subject patient, by using the first sub-prediction model, and
generate the information about the prediction result for the subject patient, based on the one or more features and the additional medical data of the subject patient, by using the second sub-prediction model.

16. The image processing system of claim 15, wherein
the processor is further configured to
generate information about a factor affecting generation of the information about the prediction result for the subject patient, by using the machine learning prediction model, and
obtain information about an importance of each of a plurality of factors in generating the information about the prediction result for the subject patient, by using the second sub-prediction model,
wherein the plurality of factors comprise at least one of the additional medical data of the subject patient or the one or more features.

17. The information processing system of claim 16, wherein
the processor is further configured to
determine at least one of the plurality of factors as a prediction reason, based on the information about the importance.

18. The image processing system of claim 15, wherein
the one or more features comprise a phenotypic feature that is usable to interpret the information about the prediction result for the subject patient.

19. The image processing system of claim 15, wherein
the first sub-prediction model is trained to extract one or more reference features based on medical image data of a reference patient, and
the second sub-prediction model is trained to generate reference information about a reference prediction result for the reference patient, based on additional medical data of the reference patient and the one or more reference features.

20. The image processing system of claim 15, wherein
the processor is further configured to
generate input data of the second sub-prediction model by concatenating the additional medical data of the subject patient with the one or more features, and
generate the information about the prediction result for the subject patient by inputting the generated input data to the second sub-prediction model.

21. The information processing system of claim 12, wherein
the additional medical data of the subject patient comprises at least one of clinical data, lab data, or biological data of the subject patient.
